(19)

Europäisches
Patentamt

European
Patent Office

Office européen
des brevets

(11) **EP 2 974 753 A1**

(12) **EUROPEAN PATENT APPLICATION**

(43) Date of publication:
**20.01.2016 Bulletin 2016/03**

(21) Application number: **14306175.2**

(22) Date of filing: **18.07.2014**

(51) Int Cl.:
***A61L 27/38*** (2006.01)          ***A61L 27/12*** (2006.01)
***A61L 27/46*** (2006.01)          ***C08L 89/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **INSTITUT NATIONAL DE LA SANTE ET DE LA RECHERCHE MEDICALE (INSERM)**
  **75013 Paris (FR)**
• **UNIVERSITE DE NANTES**
  **44000 Nantes (FR)**

(72) Inventors:
• **Gamblin, Anne-Laure**
  **22360 LANGUEUX (FR)**
• **Trichet, Valérie**
  **44270 MACHECOUL (FR)**
• **Layrolle, Pierre**
  **44300 NANTES (FR)**

(74) Representative: **Regimbeau**
  **20, rue de Chazelles**
  **75847 Paris Cedex 17 (FR)**

(54) **Bone regenerating biomaterials with selected cells from peripheral blood**

(57)     The present invention relates to a biomaterial comprising calcium phosphate particles and a cell fraction consisting essentially of isolated monocytes useful as medicament, particularly in the prevention and/or treatment of a bone loss disorder.

EP 2 974 753 A1

**Description**

The present invention relates to the field of bone regeneration.

In particular, the invention relates to a biomaterial useful for bone regeneration, particularly for the filling and regeneration of large bone loss/defects.

With more than 1 million bone graft procedures per year in Europe, bone is the most transplanted tissue in humans. There are many bone defects in patients that need to be regenerated such as non-union fractures following severe trauma, surgical revisions of orthopedic implants and spine fusions. Typically, the volume of bone defects to be regenerated ranges from 5 to 50 $cm^3$ (Balaguer et *al.*, 2010) [1]. In these medical indications, several therapeutic procedures have been proposed.

Bone defects are usually filled by an autologous bone graft. In such procedures, bone is taken from a second surgical site (e.g. iliac crest, skull, fibula) and transplanted into the defect on the same patient. The autologous bone graft contains the mineralized extracellular matrix, patient's own cells and growth factors that help bone regeneration with a satisfactory clinical success ranging from 50 % to 70 %. However, the autologous bone grafting procedure has drawbacks: the patient's bone stock is limited in quantity, it requires a second surgical site causing complications (risks of infection and fractures, pain) and its effectiveness remains limited due to significant resorption and limited regeneration capacity of the bone graft.

An alternative to transplantation of autologous bone graft is related to cell therapy, particularly tissue engineering. Classically, patient's bone marrow (10 to 50 $cm^3$) is aspirated from iliac crest under local anesthesia using heparinized syringes. Bone marrow contains a very low proportion of pluripotent cells, namely mesenchymal stem cells (MSC), able to differentiate into bone, cartilage or adipose tissues. MSC are isolated from bone marrow by, e.g., their adherence to culture treated plastic and amplified by culturing in media containing human blood derived platelet lysate (PL). Freshly harvested MSC from culture dishes are mixed with a synthetic bone substitute, namely calcium phosphate particles in the surgical room and implanted in the patient's bone defect. The inventors have shown that MSC attached on calcium phosphate particles and that this biomaterial with patient own MSC cells has osteoinductive properties and bone regeneration capacity. However, a drawback of this cell therapy is related to the very low proportion of MSC contained in bone marrow, typically 0.001 to 0.01 % of mononuclear cells (MNC). As a bone marrow aspirate contains between $15 \times 10^6$ to $30 \times 10^6$ MNC/$cm^3$, only $0.15 \times 10^3$ to $3 \times 10^3$ MSC/ $cm^3$ are present in bone marrow (Pittenger et *al.,* 1999 [2]). Consequently, MSC must be amplified in culture for several weeks to reach an effective therapeutic dose, typically $20 \times 10^6$ MSC for 1 $cm^3$ or 0.5 g of biomaterial. For clinically relevant volumes of 5 to 50 $cm^3$, the procedure requires culturing of MSC for 3 weeks in a pharmaceutical laboratory and clean room environment, many quality controls, transportation of fresh bone marrow as well as MSC suspension between the production laboratory and the operating room. Therefore, this therapeutic approach remains cumbersome and expensive for the patient.

Another bone regeneration method consists in mixing coagulated blood or bone marrow aspirates from the patient (WO 2010/007230) that contain mononucleated cells, platelets and plasma with biphasic calcium phosphate (BCP) particles having an average size between 40 to 500 $\mu$m. However, the inventors have demonstrated by comparative examples given in the present application that the bone regeneration induced with these body fluids remains significantly lower than the bone regeneration induced with the biomaterials of the present application.

Some studies have shown that cell populations containing monocytes-derived mesenchymal progenitors can differentiate into several distinct mesenchymal cell lineages such as osteogenic cells (Pignolo et *al.*, 2011 and Kuwana et *al.*, 2003) [3, 4]. However, these studies have not demonstrated the effectiveness of differentiation of these monocytes-derived mesenchymal progenitors into osteogenic cells *in vivo* in aim to be used in bone regeneration therapy.

Other authors (Mouline et *al.*, 2010) have proposed alternative strategies based on the co-culture of macrophages/monocytes and osteoprogenitor cells *in vitro* onto calcium phosphate biomaterials before transplantation. However, this approach requires extensive culturing steps to amplify and differentiate cells *in vitro* prior to implantation in patients. Furthermore, this study has shown only the differentiation of monocytes into multinucleated osteoclasts. This differentiation demands 5 days of culture with the addition of growth factors, like macrophage-colony stimulating factor and receptor activator of nuclear factor kappa-B ligand. Thus, this protocol retains the drawbacks of long cell culturing steps.

Others have hypothesized that by combining biphasic calcium phosphate (BCP) granules with autologous platelet rich plasma (PRP) gel, bone regeneration would then be improved (Rabillard et *al.,* 2009) [6]. However, the study failed to demonstrate bone healing at 16 weeks in critical size defects in ulna of dogs. No significant ossification was present, neither with BCP granules alone nor in association with PRP gel.

Another solution to accelerate bone repair have been proposed by Zhang et al. [7]. Thus, the authors have demonstrated that human monoosteophils, which are generated by cathelicidin-derived peptide LL-37 treated monocytes, mixed with calcium phosphate material (Zhang et al. 2009) [8] induce bone generation in injured sites of NOD/SCID mice. In contrast, bone formation was not observed in the control mice subcutaneously implanted with 40 mg of calcium phosphate material mixed and $5 \times 10^6$ monoosteophils alone or with calcium phosphate material mixed with LL-37 alone after 7 weeks. This study corroborated that a high dose of monocytes in combination with calcium phosphate granules is detrimental for bone tissue formation.

Another proposed strategy is to use polymer gels like alginate to trigger the activity of host macrophages/monocytes *in situ* on implantation (Dong et al., 2013) [9] Nevertheless, it is only a hypothesis and as being illustrated in the present application, the inventors have demonstrated that such strategy does not work. Indeed, monocytes encapsulated in alginate gels or mixed with microfibrous poly-E-caprolactone failed to form bone tissue in muscles of nude mice after 8 weeks.

There remains therefore a significant need for new and improved products for bone regeneration, which are: effective, cheap, quick and easy to produce, easy to insert into subjects and biocompatible. The present inventors have made a significant step forward with the invention disclosed herein.

The purpose of the invention is to fulfill the need of an easy accessible bone graft material by providing innovative bone regenerating biomaterials with selected cells, preferably from peripheral blood of the patient, which makes it possible to solve in whole or part the above mentioned problems, in particular the pre-culturing steps.

Unexpectedly, the inventors have demonstrated that the combination of calcium phosphate particles with selected monocytes isolated from peripheral blood lead to improved bone formation and bone regeneration capacities as compared to direct combination of whole blood with calcium phosphate particles similarly to the one proposed before (WO 2010/007230).

A major advantage of the present invention lies in the easy accessibility of peripheral blood as source of monocytes.

These results are surprising since monocytes are precursors of the inflammatory cells macrophages and they are strongly involved in inflammatory response. It is known in the art that high inflammation leads to fibrosis and that decreasing of inflammation, particularly at the injury site, is highly desired in bone regeneration method.

Interestingly, there are optimal doses of monocytes in unison with calcium phosphate particles to induce bone regeneration. The present invention demonstrates that biomaterials with monocytes at low concentration, i.e. such as that found in peripheral blood (4 to 11 % of the peripheral blood mononuclear cells (PBMC), typically ranging from $0.20 \times 10^6$ to $0.95 \times 10^6$ monocytes per $cm^3$ of blood), have a limited bone regenerative capacity. On the opposite, increasing the dose of monocytes above the optimal amount in the biomaterials led to acute inflammation and fibrosis and not to bone tissue regeneration.

In one aspect, the invention relates to a biomaterial comprising:

- calcium phosphate particles having a diameter from 0.2 mm to 4 mm, particularly from 0.3 to 2 mm, more particularly from 0.5 mm to 1 mm; and
- a cell fraction consisting essentially of isolated monocytes;

wherein the number of isolated monocytes per 1 g of calcium phosphate particles ranges from $0.2 \times 10^6$ to $50 \times 10^6$, particularly from $1 \times 10^6$ to $10 \times 10^6$, and more particularly from $3 \times 10^6$ to $5 \times 10^6$.

In the preferred embodiment of the invention the optimal dose found by intramuscular implantation in nude mice is $0.14 \times 10^6$ monocytes / 35 mg of BCP, i.e. $4 \times 10^6$ monocytes per 1 g of calcium phosphate.

The biomaterial according to the invention has in particular the following advantages:

- It has got a strong bone regeneration efficiency when monocytes are sorted from peripheral blood of the patient and mixed with BCP particles in the appropriate ratio.
- In the patent application WO2010/007230, bone regeneration is claimed when coagulated blood from the patient is mixed with calcium phosphate particles. However, mixing blood with synthetic calcium phosphate bone substitutes is a standard practice for surgical handling and the blood/BCP mixture has proved lower efficacy in bone regeneration of critical size defects compared to the biomaterial of the present invention. Furthermore, the number of monocytes in blood is around $0.5 \times 10^6$ monocytes per $cm^3$. This number is four-fold below the efficient dose of $2 \times 10^6$ monocytes per $cm^3$ or $4 \times 10^6$ monocytes per g of calcium phosphate particles as determined in the present invention.
- Platelet rich plasma and platelet lysates have been described in several publications as osteoinductive materials (Balaguer et al., 2010 and Rabillard et al., 2009) [1, 6]. Despite these compounds contain significant quantities of growth factors such as vascular endothelial growth factor, basic-fibroblast growth factor, etc., there are controversy reports in the literature regarding their efficacy in bone regeneration [1, 6].
- It has no severe side effects since blood sampling is a benign and medical procedure in contrast to bone harvesting;
- It has constant and homogeneous properties since the given examples have been done with several human donors with high consistency in bone regeneration;
- It avoids the drawbacks of autologous bone transplantation as well as those of MSC that require culturing for several weeks prior to surgery. In the present invention, a blood sample of 10 to 100 $cm^3$ allows isolating in few hours between $5 \times 10^6$ to $50 \times 10^6$ monocytes. This amount of monocytes is superior to the therapeutic dose of $4 \times 106$

monocytes per g of calcium phosphate granules. For clinical bone defects ranging from 5 to 20 cm$^3$, i.e. 2.5 to 10 g of calcium phosphate particles, respectively, the dose of cells should range from $10\times10^6$ to $40\times10^6$ monocytes can be isolated from a patient blood sample of 20 to 100 cm$^3$. Since monocytes can be easily cryopreserved after isolation, it is possible to harvest blood samples from the patient at different time intervals or perform cytapheresis prior to the bone regeneration surgery.

Isolation of cell fraction consisting essentially of monocytes is usually performed by blood transfusion centers that have the expertise of magnetic cell sorting from peripheral blood by using specific cell surface antibodies such as CD14 attached to magnetic beads. Normally, the preparation of said cell fraction from blood of the patient is performed within 2 to 4 hours under Good Manufacturing Practices. As the blood transfusion units are located near to the surgical rooms in hospitals, the procedures described in the present invention are compatible with common bone regeneration surgery. Nevertheless, if a high number of monocytes is needed or risks with blood collection are present, it is possible to perform cytapheresis and cryopreserve monocytes in liquid nitrogen for long periods prior to surgery.

The present invention is easy, quick and inexpensive to produce. It avoids cells culture, and thus decreases the risk of contamination. It can be obtained from a simple blood sample of a patient obtained several hours before its delivery to said patient. It avoids the drawbacks associated with the collection of samples of bone marrow, including pains, infection risk, local anesthesia. It avoids the patient coming several times to the hospital for the preparation of the biomaterial and its delivery to said patient, and eventually that the patient undergoes several anesthesia.

Unless defined otherwise, all technical and scientific terms used herein have the same meaning as commonly understood to one skilled in the relevant art.

For convenience, the meaning of certain terms and phrases employed in the specification, examples and claims are provided.

As used herein, the term "biomaterial" refers to any material, composition that is biocompatible, in particular designed to interact with biological systems, more particularly that is adequate for human body reconstruction.

As used herein, the term "calcium phosphate particles" refers to compound with a molar ratio Ca/P, preferably ranging from 1.5 to 1.67.

Synthetic calcium phosphate materials are calcined and sintered between 800 °C to 1400 °C with organic pore makers to produce porous ceramics. Synthetic materials are preferred to animal or human bone in order to avoid transmission of pathogens or immunological reaction.

According to a particular embodiment of the biomaterial of the invention, at least one of said calcium phosphate particles, particularly at least 10%, at least 20%, at least 30%, at least 40%, at least 50%, at least 60%, at least 70%, at least 80%, at least 90%, at least 99% and more particularly 100% by weight of said calcium phosphate particles based on the total weight of said calcium phosphate particles comprised in said biomaterial, comprises at least one compound selected from the group consisting of hydroxyapatite, tricalcium phosphate including beta-tricalcium phosphate, alpha-tricalcium phosphate, fluorapatite, calcium deficient apatite, octacalcium phosphate, dicalcium phosphate anhydrous or dihydrate, particularly hydroxyapatite and beta-tricalcium phosphate.

Hydroxyapatite and tricalcium phosphate (in particular beta-tricalcium phosphate) have the advantages to be bioresorbable and osteoconductive.

As used herein, the term "hydroxyapatite" refers to the compound also called hydroxylapatite, of formula $Ca_5(PO_4)_3(OH)$ (Zhang et al., 2007) [10].

As used herein, the term "beta-tricalcium phosphate" refers to the compound of formula $Ca_3(PO_4)_2$ (Jarcho et al., 1986) [11].

As used herein, the term "fluorapatite" refers to the calcium fluorophosphate compound having the formula $Ca_5(PO_4)_3F$ or the formula $Ca_{10}(PO_4)_6F_2$.

According to a particular embodiment of the biomaterial of the invention, at least one of said calcium phosphate particles, particularly at least 10%, at least 20%, at least 30%, at least 40%, at least 50%, at least 60%, at least 70%, at least 80%, at least 90%, at least 99% and more particularly 100% by weight of said calcium phosphate particles based on the total weight of said calcium phosphate particles comprised in said biomaterial, comprises hydroxyapatite and beta-tricalcium phosphate, particularly the weight ratio of said hydroxyapatite to said beta-tricalcium phosphate is from 0 to 100, more particularly from 15:85 to 25:75, even more particularly 20:80 to 30:70 and even more particularly from 40:60 to 50:50 .

Calcium phosphate particles comprising hydroxyapatite and beta-tricalcium phosphate have the advantage of having similar compositions of the mineral of bones and thus, being well approved as synthetic bone substitutes for filling bone defects.

The weight ratio of 20:80 of said hydroxyapatite to said beta-tricalcium phosphate has the advantage to provide with an optimal balance between resorption and re-precipitation of carbonated calcium deficient apatite on its surface from body fluids.

According to a particular embodiment of the biomaterial of the invention, at least one of said calcium phosphate particles, particularly at least 10%, at least 20%, at least 30%, at least 40%, at least 50%, at least 60%, at least 70%, at least 80%, at least 90%, at least 99% and more particularly 100% by weight of said calcium phosphate particles based on the total weight of said calcium phosphate particles comprised in said biomaterial, comprises macro-pores, in particular open pores, particularly interconnected pores, having a size from 10 to 1000 $\mu$m, particularly from 20 to 800 $\mu$m and more particularly from 50 to 400 $\mu$m.

Particularly, at least one of said calcium phosphate particles, particularly at least 10%, at least 20%, at least 30%, at least 40%, at least 50%, at least 60%, at least 70%, at least 80%, at least 90%, at least 99% and more particularly 100% by weight of said calcium phosphate particles based on the total weight of said calcium phosphate particles comprised in said biomaterial, comprises micro-porosity or micro-pores in the size range of 0.01 to 10 $\mu$m, particularly from 0.05 to 5 $\mu$m and more particularly from 0.1 to 2 $\mu$m. The micro-porosity at the surface of calcium phosphate ceramics is obtained by incomplete sintering.

Micro-porous phosphate calcium particles have the advantage of favoring adsorption of proteins and attachment of cells from body fluids.

Calcium phosphate particles comprising interconnected pores in the size range of 50 to 400 $\mu$m have the advantage to allow permeability of body fluids, vascularization and bone tissue ingrowth. Inter particular spaces between particles provide also with interconnected macro-porosity.

As used herein the terms "cell fraction essentially consisting of isolated monocytes" or "hMonocyte fraction" or "hMonocyte CD14+ fraction" refer to cell fraction essentially consisting of monocytes which have been isolated from at least one sample of at least one subject and thus separated from the majority of the other components of said sample(s).

Thus, according to present invention "essentially consisting of" means that specific further components can be present in cell fraction, namely those not materially affecting essential characteristics of isolated monocytes".

The purity of isolated monocytes in said cell fraction the present invention is at least 90 % of total cells. In particular, the biomaterial according to the invention, further comprising:

- less than 10 % of erythrocytes per 1 g of calcium phosphate particles; and/or
- less than 10 % of thrombocytes per 1 g of calcium phosphate particles; and/or
- less than 10 % of other peripheral blood mononuclear cells per 1 g of calcium phosphate particles; and/or
- less than 10% of mesenchymal stem cells per 1 g of calcium phosphate particles.

The cell fraction consisting essentially of isolated monocytes can be obtained by different methods well known by one skilled in the art, including flow cytometry sorting and magnetic cell sorting, performed from different types of samples including blood sample, bone marrow sample, a sample of peripheral blood mononuclear cells. Particularly, said cell fraction can be obtained according to step (i) of the method for preparing a biomaterial according to the invention. For example, the cell fraction consisting essentially of isolated monocytes can be obtained according to the method illustrated in the Examples of the present application.

As used herein the term "isolated monocytes" refers to monocytes which are isolated with said cell fraction consisting essentially of isolated monocytes.

The invention also relate to a biomaterial, wherein said calcium phosphate particles and said cell fraction are entrapped in a blood plasma fibrin clot.

According to one embodiment of the invention, the cell fraction consisting essentially of isolated monocytes is previously suspended in blood plasma supplemented with an anticoagulation agent selected from the group of calcium ions Ca$^{2+}$ chelating agents comprising sodium citrate, CTAD (mixture of Citrate, Theophylline, Adenosine and Dipyridamole), EDTA (Ethylene Diamine Tetra Acetic acid), CPD (Citrate Phosphate Dextrose)

According to the invention, the cell fraction consisting essentially of isolated monocytes is mixed with calcium phosphate particles and fibrin and treated with an excess of calcium in order to form a fibrin clot. The fibrin clot ensures that non-adherent monocytes are entrapped between calcium phosphate particles and facilitates the handling of the biomaterial. However, the fibrin clot is not a requirement in order to form a bone regenerating biomaterial and simple mixtures of the cell fraction consisting essentially of isolated monocytes and calcium phosphate particles are effective to induce bone regeneration.

The invention also relates to the biomaterial according to the invention, for use as a medicament, particularly a bone-generating medicament. The advantageous embodiments are as defined above.

As used herein, the term "bone-regenerating medicament" refers to any medicament which is useful for enhanced bone tissue engineering.

The invention also relates to the biomaterial according to the invention for use as a medicament, particularly a bone-regenerating medicament, in the prevention and/or treatment of a bone loss disorder.

A bone regenerating biomaterial is required in the following clinical situations:

- Fusion of vertebra (para spinal and/or filling of intervertebral fusion cages;

- Non-union or delayed union long-bone fractures;
- Revisions of articular joints (hip and knee articular prostheses);
- Reconstruction of large cranial or maxillofacial defects following severe trauma (e.g; ballistic, car accident) or surgical resection of bone tumors.

As used herein, a "bone loss disorder" is any condition wherein the bone mass density and/or volume is reduced. Such disorders include those in which bone loss is a primary or secondary symptom of said disorder. Disorders include diseases with an underlying pathological disturbance relative to a normal individual which may result for example from infection or an acquired or genetic imperfection. Disorders also include those resulting from intentional or unintentional ingestion of certain agents, e.g nutritionally associated conditions or alcoholism. Disorders however also include conditions generally considered to be normal, e.g. through the ageing process, or conditions attributable to physiological or surgical events, e.g. menopause, pregnancy or ovariectomy, respectively, but which exhibit undesirable bone loss. Particularly, the bone loss disorder can be selected from the group consisting of rheumatoid arthritis, periodontitis, osteoporosis, Paget's disease, hypercalcaemia of malignancy, metastatic bone disease, multiple myeloma, osteogenesis imperfecta, osteomalacia, hyperparathyroidism and hypoparathyroidism.

The biomaterial according to the invention can be delivered at a site wherein a bone generation is needed in a subject in an amount sufficient to generate bone at said site, in particular within the range from 5 to 50 cm$^3$. Said amount will be adjusted according to the quantity of bone to be generated.

The biomaterial according to the invention can be delivered at a site wherein a bone generation is needed in a subject, particularly by a surgical intervention as illustrated in the Examples of the present application. For example, after incision, the biomaterial is implanted and the incision is then closed.

The invention also relates to the use of the biomaterial according to the invention for the preparation of a medicament, particularly a bone-regenerating medicament, in particular for the prevention and/or treatment of a bone loss disorder. The advantageous embodiments are as defined above.

The invention also relates to the biomaterial according to the invention, for use in a method of bone generation in a subject, particularly wherein:

- the biomaterial is to be delivered at a site wherein a bone regeneration is needed.

The advantageous embodiments are as defined above.

The invention also relates to a method for preparing a biomaterial, particularly according to the invention, comprising the step of:

   (ii) adding calcium phosphate particles as defined above to the cell fraction consisting essentially of isolated monocytes; and

wherein the number of isolated monocytes per 1 g of calcium phosphate particles ranges from $0.2 \times 10^6$ to $50 \times 10^6$ particularly from $1 \times 10^6$ to $10 \times 10^6$ and more particularly from $3 \times 10^6$ to $5 \times 10^6$.

Step (ii) can be performed by contacting and mixing calcium phosphate particles as defined above to the cell fraction consisting essentially of isolated monocytes

In a particular embodiment, the method according to the invention, further comprises, before step (ii), the step of:

   (i) recovering the cell fraction consisting essentially of isolated monocytes from a biological sample of at least one subject.

Step (i) of recovering the cell fraction consisting essentially of isolated monocytes can be performed by any method well known by one skilled in the art, including flow cytometry sorting, magnetic cell sorting (Pickl et al., 1996; de Baey et al., 2000; Salio et al., 2000; Ebner et al., 2002; Jefford et al., 2003; Matsumoto et al., 2003; Hanley et al., 2004; Verreck et al, 2004; Rayan et al., 2002 and Vitale et al., 2004) [12, 13, 14, 15, 16, 17, 18, 19, 20, 21], performed from different types of samples including blood sample, bone marrow sample, a sample of peripheral blood mononuclear cells.

Particularly, step (i) recovering the cell fraction consisting essentially of isolated monocytes from a biological sample, particularly a sample of peripheral blood mononuclear cells of at least one subject is performed by magnetic cell sorting.

As used herein, the term "magnetic cell sorting" refers to method for separation of various cell populations depending on their surface antigens (CD molecules).

Magnetic cell sorting can be performed using at least one antibody directed against at least one specific surface antigen of monocytes, said antibodies can be bound to magnetic beads or recognized by another antibody bound to magnetic beads and directed against said antibodies. Said antibodies reacting to specific surface antigen of monocytes are used to specifically capture monocytes. This approach allows the selective enrichment of monocytes.

As used herein, the term "flow cytometry sorting" refers to Fluorescence-activated cell sorting (FACS). It provides a method for sorting a heterogeneous mixture of biological cells into two or more containers, one cell at a time, based upon the specific light scattering and fluorescent characteristics of each cell. It is a useful scientific instrument as it provides fast, objective and quantitative recording of fluorescent signals from individual cells as well as physical separation of cells of particular interest.

For example, the cell fraction consisting essentially of isolated monocytes can be obtained by magnetic cell sorting using at least one antibody directed against at least one specific surface antigen of monocytes (including antibodies directed against the CD14 antigen and/or the CD11b antigen), performed on a sample of peripheral blood mononuclear cells; said sample can be obtained by centrifugation of a blood sample.

Particularly, the cell fraction consisting essentially of isolated monocytes can be obtained according to the method illustrated in the Examples of the present application.

As used herein, the term "biological sample" means a sample of blood from the patient, typically less than 500 ml of blood.

In particular, said biological sample is selected from the group consisting of blood sample, bone marrow sample, a sample of peripheral blood mononuclear cells.

As used herein, the term "subject" refers to mammals in particular, the mammals are selected in the group comprising, human being, dogs, cats, horses, caws, sheep, goats, rats, mice, more particular the subject is human being.

The invention also relate to the method for preparing a biomaterial according to the invention further comprising, before step (ii) and after step (i), the steps of:

(I') suspend said cell fraction consisting essentially of isolated monocytes in blood plasma supplemented with an anticoagulation agent selected from the group of calcium ions $Ca^{2+}$ chelating agents comprising sodium citrate, CTAD, EDTA;

(I") adding a solution of $CaCl_2$, $Ca(NO_3)_2$, $Ca(AcOEt)_2$, or $CaSO_4$, to the monocytes/plasma solution obtained in step (I').

Since monocytes are non-adherent cells, the above method allow entrapping monocytes and calcium phosphate granules within a fibrin clot. This procedure also facilitates surgical handling of the bone regenerating biomaterial for filling complex shaped bone defects.

In the method of the present invention the cell fraction consisting essentially of isolated monocytes are suspended in blood plasma supplemented with an anticoagulation agent (sodium citrate, CTAD, EDTA). A solution of $CaCl_2$ at 200 mM is added to the monocytes / plasma solution in a 1:10 volume ratio in order to get a final concentration of 20 mM. The mix is added to the calcium phosphate granules while clotting occurred within 30 minutes. The hybrid composite can be used to fill a bone defect.

The invention also relates to a method of bone generation in a subject comprising the step of:

b) delivering the biomaterial according to the invention at a site wherein a bone regeneration is needed in the subject.

The method of bone regeneration according to the invention can comprise, prior to step b), the step of:

a) preparing a biomaterial according to the methods of the invention;
particularly wherein step (i) of recovering the cell fraction consisting essentially of of isolated monocytes is performed from a biological sample of said subject (therefore optimizing the biocompatibility of the biomaterial).

Said biomaterial can be delivered in an amount sufficient to regenerate bone at said site.

The invention also relates to a method for the treatment of a bone loss disorder, comprising the step of:

- administering to a subject in need thereof a therapeutically effective amount of a biomaterial according to the invention, particularly at a site wherein a bone generation is needed.

The method for the treatment of a bone loss disorder according to the invention can comprise, prior to the biomaterial administration step, the step of:

- preparing a biomaterial according to the methods of the invention;

Particularly wherein step (i) of recovering the cell fraction consisting essentially of isolated monocytes is performed from a biological sample of said subject (therefore optimizing the biocompatibility of the biomaterial).

The invention also relates to a medical device comprising the biomaterial according to the invention.

As used herein, the term "medical device" refers to any instrument, apparatus, appliance, material or other article, alone or in combination, used specifically for diagnostic and/or therapeutic purposes. In particular, the medical device is selected from the group consisting of any types of syringes and/or tubes.

In specific embodiment of the present invention a syringe containing calcium phosphate particles is connected to a transfusion bag containing the cell fraction consisting essentially of isolated monocytes separated by magnetic sorting of monocytes patient's blood and plasma. The calcium phosphate particles and the separated monocytes may be mixed in the syringe.

The invention also relates to a kit comprising :

- at least one antibody directed against at least one specific surface antigen of monocytes; and
- calcium phosphate particles as defined above.

Particularly, the kit according to the invention can comprise at least one antibody bound to magnetic beads and that is directed against at least one specific surface antigen of monocytes

Particularly, said at least one antibody directed against at least one specific surface antigen of monocytes can be bound to magnetic beads.

Particularly, said at least one antibody directed against at least one specific surface antigen of monocytes is selected from the group consisting of antibodies directed against the CD14 antigen, antibodies directed against the CD11b antigen, particularly antibodies directed against the human CD14 antigen, antibodies directed against the human CD11b antigen.

**BRIEF DESCRIPTION OF DRAWINGS**

Figure 1 shows the principle of magnetic cell sorting of monocytes from peripheral blood,

Figure 2 shows a flow cytometry of PBMC and CD14[+];

Figure 3 shows SEM photographs of BCP of 0.5 to 1mm in size (figure 3a) with microporous surface (figure 3b);

Figure 4 shows the histology of intramuscular implants of BCP, hMSC/BCP and h-monocytes/BCP after 1, 2, 4 and 8 weeks in nude mice (Masson Trichrome stain);

Figure 5 shows a histology showing ectopic bone tissue formation after implantation in muscles of nude mice for 8 weeks of BCP granules, hMSC/BCP and h-Monocytes/BCP (Masson Trichrome stain);

Figure 6 shows a histomorphometry results after implantation in muscles of nude mice with BCP alone, BCP+h-monocytes CD14+ or BCP+hMSC at 1, 2, 4 and 8 weeks. ;

Figure 7 shows a regeneration of critical size defects (4 mm) in calvaria of nude mice after 8 weeks;

Figure 8 shows a quantitative intramuscular bone formation of BCP particles with hMSC, blood clot and different doses of CD14[+] monocyte cells;

Figure 9 shows histology of BCP particles with hMSC, blood clot and different doses of CD14[+] monocyte cells after intramuscular implantations in nude mice for 8 weeks (Masson Trichrome stain);

Figure 10 shows a quantitative calvaria bone formation of BCP particles with PRP, blood clot, PBMC and different doses of CD14[+] monocyte cells;

Figure 11 shows histology of calvaria bone defect regeneration in nude mice with BCP and different doses of CD14[+] monocytes cells after 8 weeks (Masson Trichrome stain),

Figure 12 shows histology of intra muscular bone formation with CD14[+] monocyte cells and different biomaterials (alginate gel ; microfibers of poly-ε-caprolactone) after 8 weeks in nude mice (Masson Trichrome stain).

The present invention will be explained in detail with examples in the following text, but the technical scope of the present invention is not limited to those examples.

## EXAMPLES

### I. Materials and Methods

#### I.1 Biomaterial

Biphasic calcium phosphate (BCP) particles sizing between 0.5 and 1 mm were obtained from Biomatlante. The BCP particles were composed of 80 % beta-tricalcium phosphate (TCP; $Ca_3(PO_4)_2$) and 20 % hydroxyapatite (HA; $Ca_{10}(PO_4)_6(OH)_2$). The BCP particles were weighted in aliquots of 35 mg and sterilized by autoclaving at 121 °C for 20 min. BCP discs (diameter, 7 mm; thickness, 2 mm) were used for calvaria bone regeneration. The overall porosity (% v/v) of both BCP particles and discs was $75 \pm 5$ %, with a pore size distribution of 70 % (0-10 $\mu$m), 20 % (10-100 $\mu$m) and 10 % (100-300 $\mu$m).

#### I.2 Magnetic sorting of human monocytes (h-Monocytes) from peripheral blood

Citrated peripheral blood samples from healthy human donors were diluted with the same volume of phosphate buffer saline pH 7.4 (PBS) and then centrifuged under density gradient (Biocoll separating solution, 1.077 g/mL; Biochrom) to recover the PBMC. After two washes with PBS, PBMC were re-suspended at $125.10^6$ cells/mL in separation buffer (0.5 % w/w bovine serum albumin and 2 mM EDTA in PBS) and anti-human-CD14 microbeads (Miltenyi Biotec) were added according to the supplier instructions. After positive cell sorting using the MACS system (Miltenyi Biotec) according to manufacturer's instructions, human CD14$^+$ (hCD14) cells were harvested and stored at -80 °C in foetal calf serum (Sigma-Aldrich) supplemented with 10 % dimethylsulfoxide (DMSO; Sigma-Aldrich).

#### I.3 Isolation and culture of human MSC from bone marrow

Human bone marrow derived mesenchymal stem cells (hMSC) were provided by the German blood transfusion centre (IKT, University of Ulm). After receiving informed consent from healthy donors, 30 mL of bone marrow were aspirated using heparinized syringe under local anesthesia on the posterior iliac crest. Serology testing (VIH1, VIH2, VHB, VHC and Syphilis) was done on each donor for safety. Bone marrow MNC were counted, seeded at of $5.10^4$ cells/cm$^2$ on culture treated polystyrene flasks (Cellstack, Corning), and cultured in $\alpha$MEM (Gibco) supplemented with 5 % human platelet lysate (PL, IKT) and 1 IU/mL heparin (Choay) at 37 °C and 5 % $CO_2$. After 14 days of culture, hMSC were detached using trypsin/EDTA (Life Technologies), counted and seeded at $1.10^4$ cells/cm$^2$ on new cell culture flasks and cultured in $\alpha$MEM supplemented with 8 % PL for 7 days. Finally, hMSC were harvested by trypsin/EDTA and washed in PBS. For each batch, several controls were done upon delivery: cell viability $\geq$ 80 % by trypan blue exclusion; hMSC identity (> 80 % CD90$^+$, CD73$^+$ and CD105$^+$ cells, and < 5 % CD45$^+$ and HLAcII$^+$ cells) by flow cytometry analysis.

#### I.4 Evaluation of bone regeneration properties of the biomaterials in vivo

All animal experimentations were conducted according to the European guidelines after receiving approvals from the local ethic committee (agreement No. 2012.27 and 2012.198). Immune compromised female nude mice (4 weeks old, Rj:NMRI-nu; Janvier Labs) were used. Mice were housed with water and food *ad libitum* and acclimated for at least 10 days before surgery. All procedures were performed under general anaesthesia by inhalation of 2.5 % isofluorane gas (Forene). Appropriate analgesia was used by intramuscular injections of buprenorphine (Buprécare 60 $\mu$L/kg/day, Axience). Euthanasia was performed under general anesthesia by cervical dislocation. Implants were carefully retrieved after euthanasia and then processed for histology.

Ectopic bone tissue formation was tested in para-tibial muscles. Briefly, a skin incision was made to access the paratibial muscle. Muscle dissection was carefully conducted and BCP granules with or without human monocytes or MSC were implanted. Both muscle and skin were finally sutured.

To create critical size bone defects in calvaria, mice were placed on a stereostatic frame (Leica). A skin incision was made to expose the skull. A 4 mm hole was created in the calvaria bone by using a trephine and a dental hand piece (NM3000, Nouvag). Constant saline irrigation was used during drilling. After placing the implant, the skin was carefully put back to avoid motion of the implant and skin was finally sutured.

I.5 <u>Ectopic bone tissue formation in muscles of nude mice</u>

Three groups consisting of 20 mice each (n=5 animals per time point and per group) were made up: i) 35 mg BCP granules with 70 $\mu$L PBS (negative control) ; ii) 35 mg of BCP granules loaded with $1.4\times10^6$ hMSC in 70 $\mu$L PBS (positive control) ; and iii) 35 mg of BCP granules loaded with $1.4\times10^6$ hCD14 in 70 $\mu$L PBS (test material). Cells were seeded onto BCP granules 60 min prior to surgery. Each animal was bilaterally implanted with the same preparation. After 1, 2, 4, and 8 weeks, animals were euthanized.

I.6 <u>Determination of the optimal hCD14 cell dose by implantation in muscles of nude mice</u>

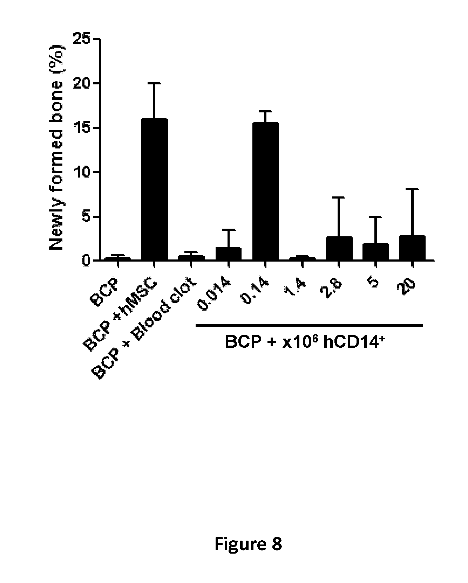

Different preparations of hCD14 with BCP granules (35 mg) in 70 $\mu$L PBS were done: $0.014\times10^6$, $0.14\times10^6$ , $1.4\times10^6$, $2.8\times10^6$ , $5\times10^6$, $10\times10^6$ (not shown), and $20\times10^6$ human CD14+ cells (figure 8). Mice were implanted bilaterally in para-tibial muscles (n=4 animals per cell dose). Euthanasia was conducted after 8 weeks implantation.

I.7 <u>Bone regeneration of critical size defects in calvaria of nude mice</u>

Implantation was carried out on 40 mice divided into 8 groups (n=5 animals each). Critical size defects of 4 mm in diameter were made on the calvaria of nude mice. BCP discs were placed on top of defects and served as negative controls. As positive control, hMSC were seeded on the BCP disc at a density of $35\times10^6$ hMSC/disc for 60 min and placed on top of the calvaria defects. hCD14+ Monocytes were seeded onto the top of a BCP disc 60 min prior to implantation and then placed over the calvaria defect. Two doses of hCD14+ cells were tested : 3.5 x $10^6$ (therapeutic dose) and 35 x$10^6$ hCD14+ cells/disc (high dose). Human blood was centrifuged on a density gradient and the PBMC were collected. Similarly, 35 x$10^6$ PBMC were seeded on BCP disc and implanted. Another group consisted of BCP discs and blood clot obtained by adding 100 $\mu$L of whole blood mixed with 20 $\mu$L CaCl$_2$ at a concentration of 200 mM. Finally, a group of platelet rich plasma (PRP) was prepared by collection of the patient's whole blood (that is anticoagulated with citrate dextrose) before undergoing two stages of centrifugation designed to separate the PRP aliquot from platelet-poor plasma and red blood cells. After 8 weeks, the mice were sacrificed and samples were processed for histology.

Table 1: *Groups of nude mice treated with BCP discs and different blood components.*

| Group | Sample description | Abbreviation |
|---|---|---|
| 1 | Empty hole of 4 mm in diameter | EMPTY |
| 2 | BCP disc 7 mm in diameter and 2 mm in thickness | BCP |
| 3 | BCP disc + $35\times10^6$ hMSC (positive control) | BCP + 35 MSC |
| 4 | BCP disc + $3.5\times10^6$ hCD14+ cells | BCP + 3.5 M |
| 5 | BCP disc + $35\times10^6$ hCD14+ cells (Monocytes high dose) | BCP + 35 M |
| 6 | BCP disc + $35\times10^6$ human PBMC | BCP + PBMC |
| 7 | BCP disc + 100 $\mu$L whole blood mixed with 20 $\mu$L CaCl$_2$ | BCP + blood clot |
| 8 | BCP disc + 100 $\mu$L platelet rich plasma PRP | BCP + PRP |

1.9 <u>Histology</u>

After euthanasia, the explants were dissected and fixed in 4 % paraformaldehyde (PFA, Microm Microtech) buffer for 24 hours. Then, the samples were decalcified in PBS solution with 0.5 % PFA and 4.13 % EDTA, and heated at 46 °C using an automated microwave decalcifying apparatus (KOS Histostation, Milestone Med. Corp.). Samples were then rinsed with tap water and dehydrated in ascending series of ethanol baths (80 %, 95 %, and 100 %), and finally in butanol for 1 hour each using an automated dehydration station (Microm Microtech). Samples were then impregnated in liquid paraffin at 56 °C (Histowax) and embedded. Thin sections of 3 $\mu$m were made perpendicular to the plane of the skin for intramuscular implants and calvaria defects using a standard microtome device (Leica RM2250). Sections were stained with Masson's trichrome technique using an automated coloration station (Microm Microtech). Stained slices were digitally scanned (NanoZoomer 2.0RS, Hamamatsu Corp.) and observed with the virtual microscope (NDP view software, Hamamatsu Corp). The bone incidence was determined as the number of samples containing bone tissue over the total number of samples. Images histomorphometry was processed on the whole implant sections using ImageJ and the percentages of biomaterial and bone tissue were calculated. The quantity of new bone was measured in separate sections with the software ImageJ. The height was reported as an average by measuring bone surface and BCP granules surface. The bone formation was evaluated as follows:

$$\% \text{ bone} = (\text{bone surface} \times 100) / (\text{total implant surface} - \text{BCP particles surface})$$

## II. Results

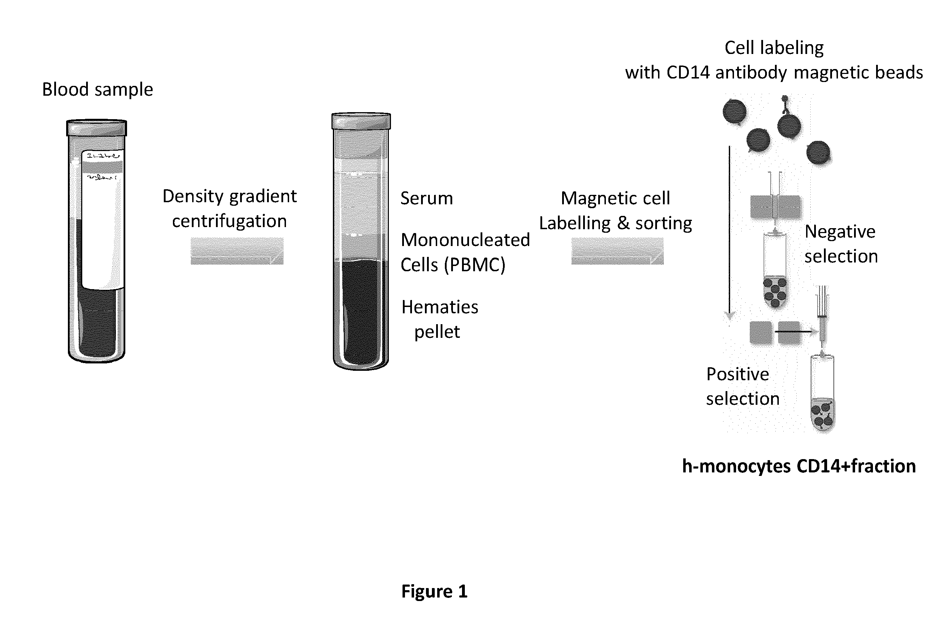

Figure 1 shows the procedure used to isolate a cell fraction consisting essentially of h-Monocytes CD14+ from patient's whole blood. A sample of patient's whole blood is collected in tubes with citrate dextrose anticoagulant or another anticoagulation agent. After gradient centrifugation of whole blood (Ficoll), the plasma is collected and the mononuclear cells fraction PBMC is recovered by pipetting in a new tube. The PBMC are diluted in PBS and monocytes are labeled with CD14 antibody attached to magnetic micro beads. By applying a magnetic field, the cell fraction consisting essentially of h-monocytes CD14+ is retained in the column while the most of other mononuclear cells such as lymphocytes are eluted. Human monocytes CD14+ cells are recovered when the magnet is removed. The method has a high yield and is rapid. For instance, a blood sample of 100 ml taken from the patient is sufficient to isolate $1 \times 10^9$ PBMC by density gradient centrifugation and to sort $200 \times 10^6$ Monocytes CD14+ by magnetic antibody labeling in a few hours.

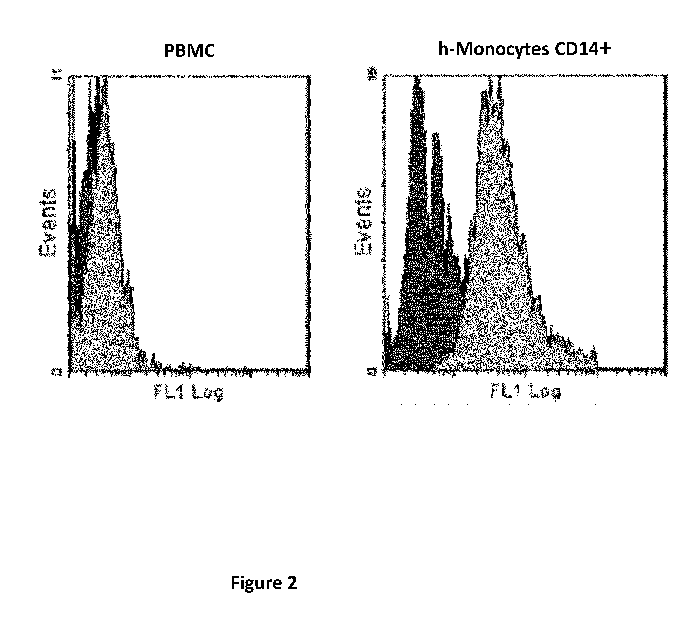

As shown in Figure 2, flow cytometry indicated that the cell population is enriched to a minum of 90 % with Monocytes CD14+ after magnetic cell sorting of PBMC.

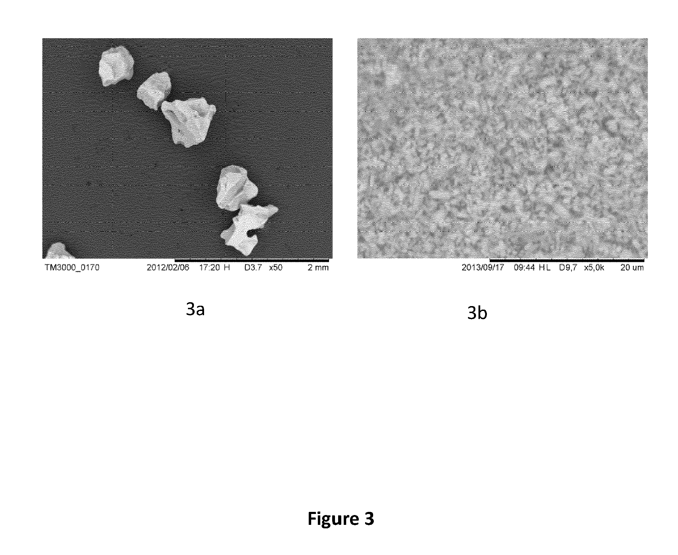

Figure 3 shows the micro porous biphasic calcium phosphate particles having average size from 0.5 to 1 mm. The BCP particles have irregular shape in order to fill and to fit the anatomy of bone defects. The spaces between the BCP granules allow a porosity and permeability of body fluids and cells as well as the ingrowth of tissues. At high magnification, the surface of BCP particles exhibits micro porosity with micropores between 0.1 to 2 $\mu$m in size. This micro porosity is due to a relatively low sintering temperature of BCP (e.g. 1050 °C).

After isolation, cell fraction consisting essentially of h-Monocytes CD14+ is suspended in patient's blood plasma at the desired cell concentration and a $CaCl_2$ solution (200 mM, volume ration 1:10) is added. The mixture is finally added to the BCP granules. After 20 min, the clotting of fibrin entrapped monocytes and calcium phosphate particles to form a paste for filling bone defects of complex shape. This time is compatible with a per surgery association of said cell fraction consisting essentially of Monocytes CD14+ and BCP granules for bone grafting. In a typical experiment, 70 $\mu$l of the said fraction wherein the Monocytes CD14+ at a cell concentration of $1.4 \times 10^6$/ml are mixed with 35 mg of BCP particles. For comparison, bone marrow derived hMSC were isolated and culture amplified for 3 weeks prior to be mixed with BCP granules by using a similar cell/biomaterial ratio.

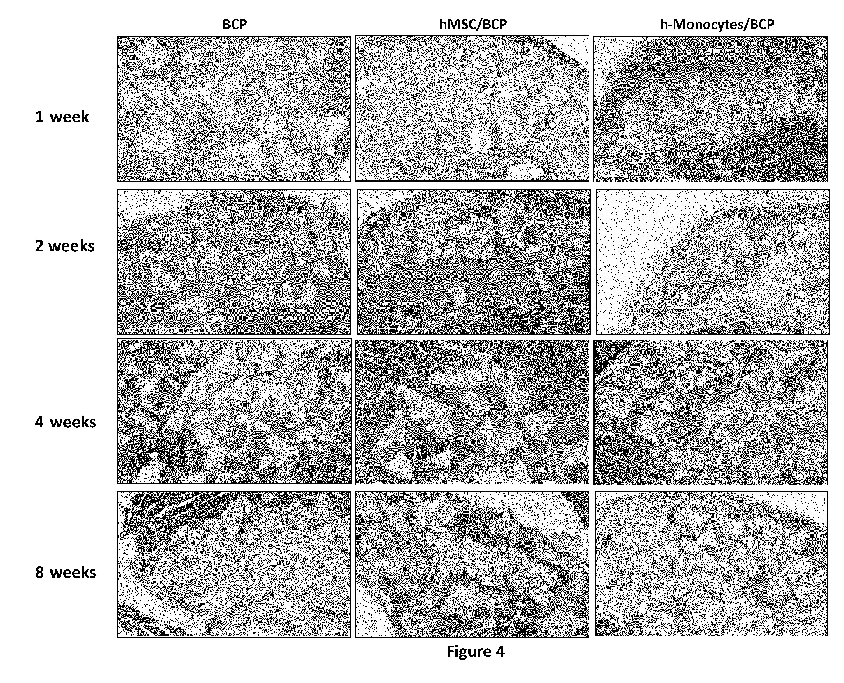

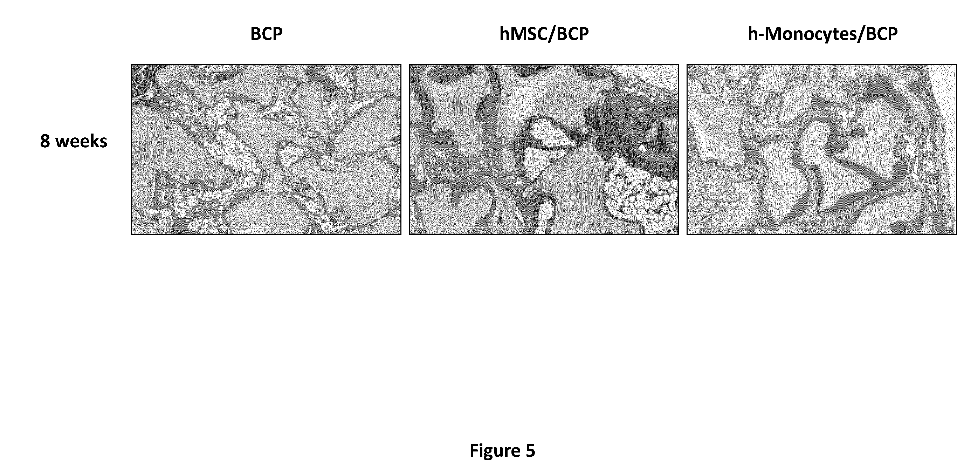

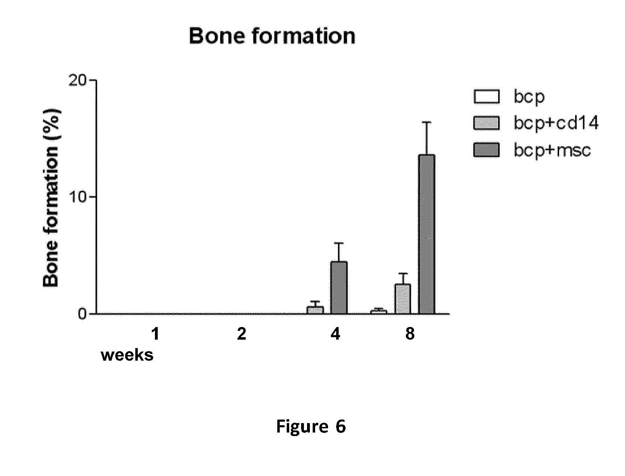

In order to demonstrate the ability of the cell/biomaterial preparation to induce bone tissue in ectopic sites, intra muscular implantations were performed in immune compromised nude mice. After 1, 2, 4 and 8 weeks, histology revealed that the negative control group consisting of BCP particles was only encapsulated by fibrous tissue until 4 weeks (figure 4). In the negative control BCP group, a sparse amount of bone tissue (<1 % of total implant surface) with a low bone incidence (2/10) was found after intramuscular implantation of BCP for 8 weeks (Figures 4, 5 and 6). In the positive control group consisting of pre-cultured hMSC mixed with BCP particles, the quantity of bone tissue was 4-5% in 6/6 at 4 weeks and 14% in 7/7 samples at 8 weeks. Nevertheless, hMSC require isolation from bone marrow and expansion in culture for 3 weeks prior to implantation. In the h-Monocytes CD14+ fraction /BCP group, a limited quantity of ectopic bone tissue (1-2%) was observed as early as 4 weeks in 3 out of 7 implanted hMonocytes fraction/BCP samples and a significant amount (3-4%) in all samples at 8 weeks (6/6). This experiment demonstrates that cell fraction consisting essentially of h-Monocytes CD14+ isolated from patient's blood and mixed with BCP particles are able to induce bone tissue in ectopic sites that are not favorable for bone tissue formation.

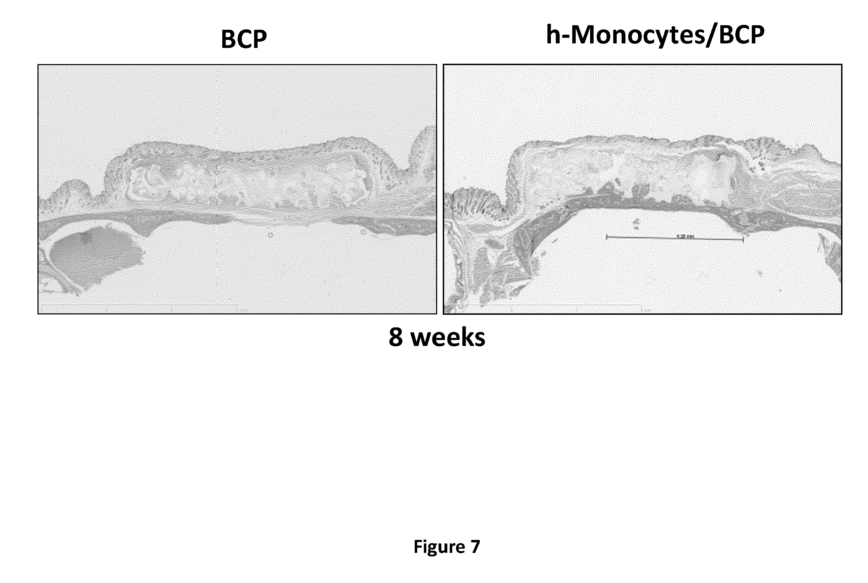

The bone regeneration capacity of h-Monocytes/BCP was tested on calvaria of nude mice. A full thickness critical size defect of 4 mm in diameter was created on the skull and was covered with BCP or h-Monocytes/BCP discs. As shown in Figure 7, the mixture of h-Monocytes/BCP had regenerated the critical size bone defect in 8 weeks while the sole BCP material has a limited effect on bone regeneration.

In another experiment, the optimal dose of monocytes to induce bone tissue was studied. The results obtained after implantation of BCP particles alone, in association of culture expanded hMSC, blood clot and different doses of CD14+ monocytes cells (figure 8) show that the optimal concentration for bone regeneration is $0.14 \times 10^6$ h-monocytes CD14+ for 35 mg of BCP particles. The bone regeneration with this concentration of monocytes is the same as bone regeneration obtained with hMSC that are cultivated for three weeks. Surprisingly, higher doses of h-Monocytes than $0.14 \times 10^6$ h-monocytes CD14+ for 35 mg of BCP were not beneficial for bone tissue formation. Furthermore, bone regeneration obtained with this concentration of monocytes is higher than the one obtained with BCP particles mixed to blood clot. These results, distinctly shown in figure 8, demonstrate that very low doses of monocytes is not sufficient to allow bone regeneration and very high doses of monocytes lead to inflammation process and worst bone regeneration.

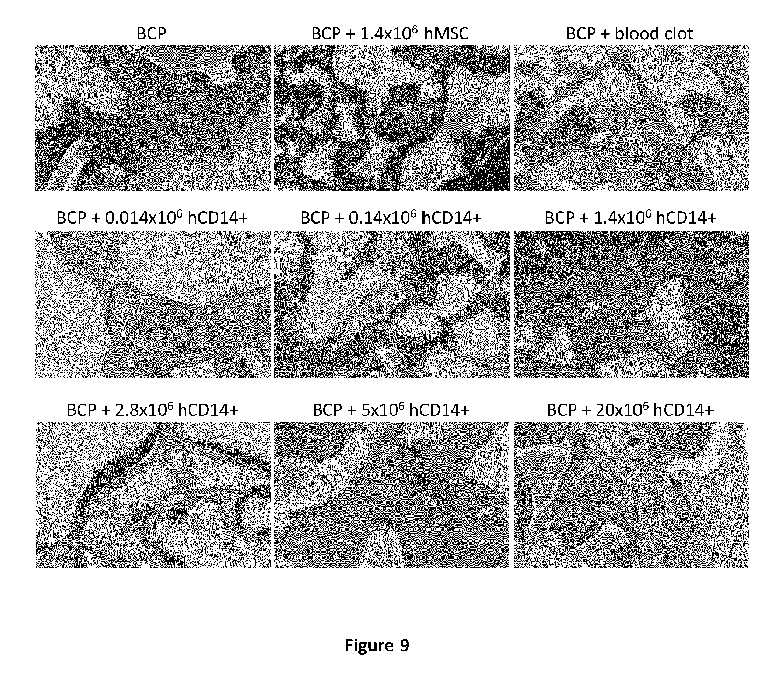

Histology pictures of BCP particles with hMSC, blood clot and differents doses of CD14+ monocytes after intramuscular implantations in nude mice for 8 weeks (Masson trichrome stain) are shown in figure 9.

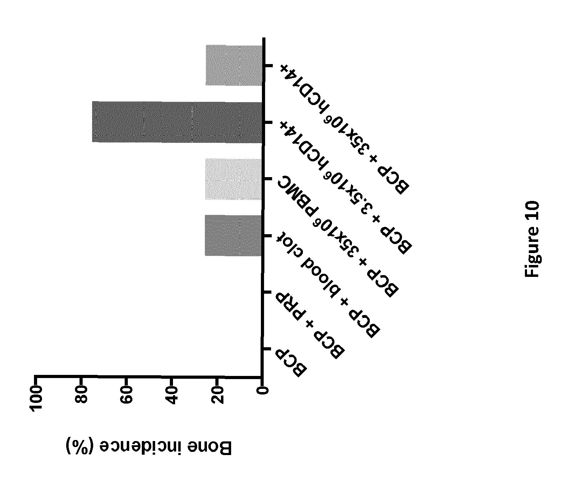

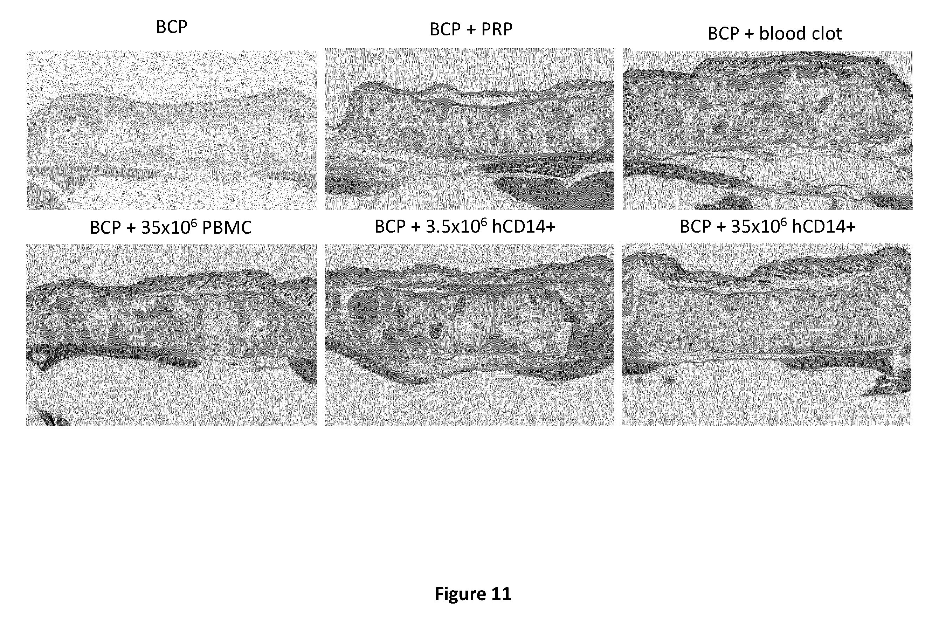

Furthermore, figures 10 and 11 show the same results for bone regeneration in calvaria defects. Bone tissue regeneration is not observed with BCP alone and BCP+PRP. Limited bone regeneration is noticed with BCP+blood clot, with a high dose of PBMC or monocytes. The optimal dosage for bone regeneration is 3.5 x $10^6$ h-Monocytes CD14+ per BCP disc corresponding to a similar dose as mentioned above for intramuscular implantations.

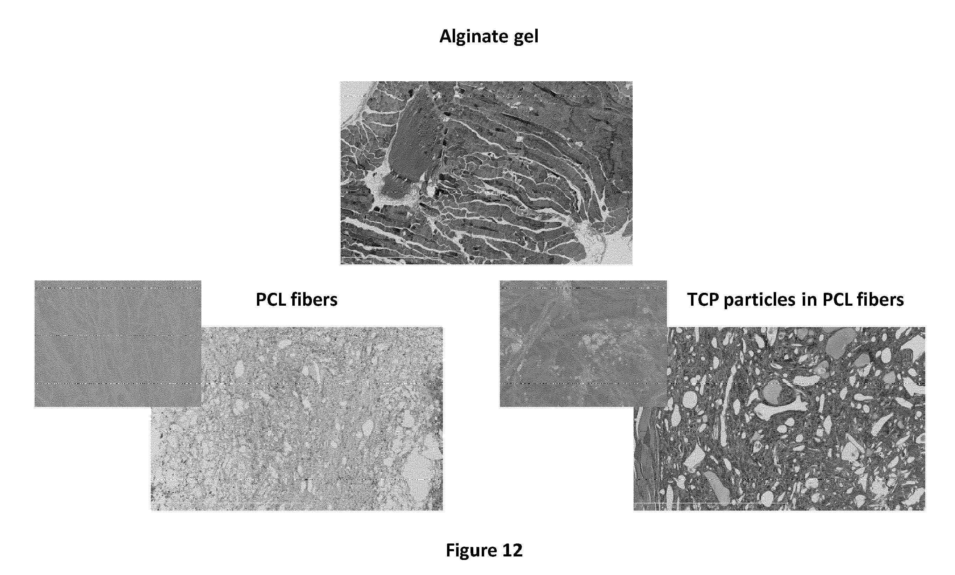

Otherwise, figure 12 shows lack of bone generation when CD14+ monocytes cell are mixed to other biomaterials such as alginate gel and poly-epsilon caprolactone (PCL) microfibers.

Therefore, the above examples demonstrate the advantages of the biomaterial of the present invention compared to the biomaterials of prior art.

## BIBLIOGRAPHIC REFERENCES

[1] T. Balaguer, F. Boukhechba, A. Clavé, S. Bouvet-Gerbettaz, C. Trojani, J. Michiels, J. Laugier, J. Bouler, G. Carle, J. Scimeca and N. Rochet, "Biphasic Calcium Phosphate Microparticles for Bone Formation: Benefits of Combination with Blood Clot," Tissue Engineering, part A, vol. 16, no. 11, pp. 3495-3505, 2010.

[2] M. Pittenger, A. Mackay, S. Beck, R. Jaiswal, R. Douglas, J. Mosca, M. Moorman, D. Simonetti, S. Craig and D. Marshak, "Multilineage potential of adult human mesenchymal stem cells," Science, vol. 284, no. 5411, pp. 143-147, 1999.

[3] R. Pignolo and M. Kassem, "Circulating Osteogenic Cells: Implications for Injury, Repair, and Regeneration," Journal of Bone and Mineral Research, vol. 26, no. 8, pp. 1685-1693, 2011.

[4] M. Kuwana, Y. Okazaki, H. Kodama, K. Izumi, H. Yasuoka, Y. Ogawa, Y. Kawakami and Y. Ikeda, "Human circulating CD14+ monocytes as a source of progenitors that exhibit mesenchymal cell differentiation," Journal of Leukocyte Biology, vol. 74, pp. 833-845, 2003.

[5] C. Mouline, D. Quincey, J. Laugier, G. Carle, J. Bouler, N. Rochet and J. Scimeca, "Osteoclastic differentiation of mouse and human monocytes in a plasma clot/biphasic calcium phosphate microparticles composite," European Cells and Materials, vol. 20, pp. 379-392, 2010.

[6] M. Rabillard, J. Grand, E. Dalibert, B. Fellah, O. Gauthier and G. Niebauer, "Effects of autologous platelet rich plasma gel and calcium phosphate biomaterials on bone healing in an ulnar ostectomy model in dogs," Vet Comp Orthop Traumatol, vol. 22, pp. 460-466, 2009.

[7] Z. Zhang and J. Shively, "Acceleration of Bone Repair in NOD/SCID Mice by Human Monoosteophils, Novel LL-37-Activated Monocytes," PLOS ONE, vol. 8, no. 7, 2013.

[8] Z. Zhang and J. Shively, "Generation of Novel Bone Forming Cells (Monoosteophils) from the Cathelicidin-Derived Peptide LL-37 Treated Monocytes," PLOS ONE, vol. 5, no. 11, 2010.

[9] L. Dong and C. Wang, "Harnessing the power of macrophages/monocytes for enhanced bone tissue engineering," Trends Biotechnol, vol. 31, no. 6, pp. 342-346, 2013.

[10] H. Zhang and Q. Zhu, "Preparation of porous hydroxyapatite with interconnected pore architecture," J MaterSci Mater Med, vol. 18, no. 9, pp. 1825-1829, 2007.

[11] M. Jarcho, "Biomaterial aspects of calcium phosphates. Properties and applications.," Dent Clin North Am, vol. 30, no. 1, pp. 25-47, 1986.

[12] W. Pickl, O. Majdic, P. Kohl, J. Stöckl, E. Riedl, C. Scheinecker, C. Bello-Fernandez and W. Knapp, "Molecular and functional characteristics of dendritic cells generated from highly purified CD14+ peripheral blood monocytes," J Immunol, vol. 157, no. 9, pp. 3850-3859, 1996.

[13] A. de Baey and A. Lanzavecchia, "The role of aquaporins in dendritic cell macropinocytosis," J Exp Med, vol.

191, no. 4, pp. 743-748, 2000.

[14] M. Salio, V. Cerundolo and A. Lanzavecchia, "Dendritic cell maturation is induced by mycoplasma infection but not by necrotic cells," Eur J Immunol, vol. 30, no. 2, pp. 705-708, 2000.

[15] S. Ebner, S. Hofer, V. Nguyen, C. Furhapter, M. Herold, P. Fritsch, C. Heufler and N. Romani, "A novel role for IL-3: human monocytes cultured in the presence of IL-3 and IL-4 differentiate into dendritic cells that produce less IL-12 and shift Th cell responses toward a Th2 cytokine pattern," J Immunol, vol. 168, no. 12, pp. 6199-6207, 2002.

[16] M. Jefford, M. Schnurr, T. Toy, K. Masterman, A. Shin, T. Beecroft, T. Tai, K. Shortman, M. Shackleton, I. Davis, P. Parente, T. Luft, W. Chen, J. Cebon and E. Maraskovsky, "Functional comparison of DCs generated in vivo with Flt3 ligand or in vitro from blood monocytes: differential regulation of function by specific classes of physiologic stimuli," Blood, vol. 102, no. 5, pp. 1753-1763, 2003.

[17] M. Matsumoto, K. Funami, M. Tanabe, H. Oshiumi, M. Shingai, Y. Seto, A. Yamamoto and T. Seya, "Subcellular localization of Toll-like receptor 3 in human dendritic cells," J Immunol., vol. 171, no. 6, pp. 3154-3162, 2003.

[18] P. Hanley, B. Musset, V. Renigunta, S. Limberg, A. Dalpke, R. Sus, K. Heeg, R. Preisig-Müller and J. Daut, "Extracellular ATP induces oscillations of intracellular Ca2+ and membrane potential and promotes transcription of IL-6 in macrophages," Proc Natl Acad Sci USA, vol. 101, no. 25, pp. 9479-9484, 2004.

[19] F. Verreck, T. de Boer, D. Langenberg, M. Hoeve, M. Kramer, E. Vaisberg, R. Kastelein, A. Kolk, R. de Waal-Malefyt and T. Ottenhoff, "Human IL-23-producing type 1 macrophages promote but IL-10-producing type 2 macrophages subvert immunity to (myco)bacteria," Proc Natl Acad Sci USA, vol. 101, no. 13, pp. 4560-4565, 2004.

[20] E. Ryan, A. Marshall, D. Magaletti, H. Floyd, K. Draves, N. Olson and E. Clark, "Dendritic cell-associated lectin-1: a novel dendritic cell-associated, C-type lectin-like molecule enhances T cell secretion of IL-4," J Immunol, vol. 169, no. 10, pp. 5638-5648, 2002.

[21] S. Vitale, A. Schmid-Alliana, V. Breuil, M. Pomeranz, M. Millet, B. Rossi and H. Schmid-Antomarchi, "Soluble fractalkine prevents monocyte chemoattractant protein-1-induced monocyte migration via inhibition of stress-activated protein kinase 2/p38 and matrix metalloproteinase activities," J Immunol, vol. 172, no. 1, pp. 585-592, 2004.

**Claims**

1. A biomaterial comprising :

   - calcium phosphate particles having an average diameter from 0.05 mm to 4 mm, particularly from 0.1 mm to 2 mm and more particularly from 0.1 mm to 1 mm; and
   - a cell fraction consisting essentially of isolated monocytes;

   wherein the number of isolated monocytes in said fraction per 1 g of calcium phosphate particles is from $0.5 \times 10^6$ to $100 \times 10^6$, particularly from $2 \times 10^6$ to $80 \times 10^6$ and more particularly from $30 \times 10^6$ to $40 \times 10^6$.

2. The biomaterial according to claim 1, said fraction further comprises:

   - less than 10 % of erythrocytes relative to the total number of cells in said fraction per 1 g of calcium phosphate particles; and/or
   - less than 10 % of thrombocytes relative to the total number of cells in said fraction per 1 g of calcium phosphate particles; and/or
   - less than 10 % of other peripheral blood mononuclear cells relative to the total number of cells in said fraction per 1 g of calcium phosphate particles; and/or
   - less than 10 % of mesenchymal stem cells relative to the total number of cells in said fraction per 1 g of calcium phosphate particles.

3. The biomaterial according to claim 1 or 2, wherein at least one of said calcium phosphate particles comprises at least one compound selected from the group consisting of hydroxyapatite, tricalcium phosphate including beta-tricalcium phosphate, fluorapatite, alpha-tricalcium phosphate, calcium deficient apatite, octacalcium phosphate, dicalcium phosphate anhydrous or dihydrate, particularly hydroxyapatite and beta-tricalcium phosphate.

4. The biomaterial according to claim 3, wherein at least one of said calcium phosphate particles comprises hydroxyapatite and beta-tricalcium phosphate, and the weight ratio of said hydroxyapatite to said beta-tricalcium phosphate is from 15:85 to 25:75, more particularly from 20:80 to 30:70 and even more particularly from 40:60 to 50:50 .

5. The biomaterial according to any one of claims 1 to 4, wherein at least one of said calcium phosphate particles comprises micro-pores, , having a size from 0.01 to 10 $\mu$m, particularly from 0.05 to 5.00 $\mu$m and more particularly from 0.1 to 2 $\mu$m.

6. The biomaterial according to any one of claims 1 to 5, wherein said calcium phosphate particles and the isolated monocytes are entrapped in fibrin clot.

7. The biomaterial according to claim 6, wherein said cell fraction consisting essentially of isolated monocytes is previously suspended in blood plasma supplemented with an anticoagulation agent selected from the group of $Ca^2$ chelates comprising sodium citrate, CTAD, EDTA and then treated with $CaCl_2$

8. The biomaterial according to any one of claims 1 to 7, for use as a medicament.

9. The biomaterial for use according to claim 8, wherein the medicament is a bone-regenerating medicament, particularly for use in the prevention and/or treatment of a bone loss disorder.

10. The biomaterial according to any one of claims 1 to 7, for use in a method of bone regeneration in a subject.

11. A method for preparing a biomaterial comprising the step of:

(ii) adding calcium phosphate particles as defined according to any one of claims 1, 3 to 5 to a cell fraction consisting essentially of isolated monocytes; and

wherein the number of isolated monocytes in said fraction per 1g of calcium phosphate particles is from $0.5 \times 10^6$ to $100 \times 10^6$, particularly from $2 \times 10^6$ to $80 \times 10^6$ and more particularly from $30 \times 10^6$ to $40 \times 10^6$.

12. The method according to claim 11, further comprising, before step (ii), the step of:

(i) recovering a cell fraction consisting essentially of isolated monocytes from a biological sample of at least one subject.

13. The method according to claim 11 and 12 further comprising, before step (ii) and after step (i), the steps of:

(I') suspending said cell fraction consisting essentially of isolated monocytes in blood plasma supplemented with an anticoagulation agent selected from the group of $Ca^2$ chelates comprising sodium citrate, CTAD, EDTA; (I") adding a solution of $CaCl_2$ to the cell fraction/plasma solution obtained in step (I').

14. The method according to claims 12 and 13, wherein step (i) of recovering cell fraction consisting essentially of isolated monocytes from a biological sample, particularly a sample of peripheral blood mononuclear cells, of at least one subject is performed by magnetic cell sorting.

15. A kit comprising :

- at least one antibody directed against at least one specific surface antigen of monocytes selected from the group consisting of antibodies directed against the CD14 antigen, antibodies directed against the CD11b antigen, particularly antibodies directed against the human CD14 antigen, antibodies directed against the human CD11b antigen ; and
- calcium phosphate particles as defined according to any one of claims 1, 3 to 5.

Figure 1

**Figure 2**

Figure 3

Figure 4

BCP          hMSC/BCP          h-Monocytes/BCP

8 weeks

Figure 5

**Figure 6**

h-Monocytes/BCP

BCP

8 weeks

Figure 7

**Figure 8**

BCP + blood clot

BCP + 1.4x10$^6$ hMSC

BCP

BCP + 1.4x10$^6$ hCD14+

BCP + 0.14x10$^6$ hCD14+

BCP + 0.014x10$^6$ hCD14+

BCP + 20x10$^6$ hCD14+

BCP + 5x10$^6$ hCD14+

BCP + 2.8x10$^6$ hCD14+

Figure 9

Figure 10

BCP

BCP + PRP

BCP + blood clot

BCP + 35x10$^6$ PBMC

BCP + 3.5x10$^6$ hCD14+

BCP + 35x10$^6$ hCD14+

Figure 11

Figure 12

Europäisches
Patentamt

European
Patent Office

Office européen
des brevets

# EUROPEAN SEARCH REPORT

**Application Number**

EP 14 30 6175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. BERNHARDT ET AL: "Nanocrystalline spherical hydroxyapatite granules for bone repair: in vitro evaluation with osteoblast-like cells and osteoclasts", JOURNAL OF MATERIALS SCIENCE: MATERIALS IN MEDICINE, vol. 24, no. 7, 28 April 2013 (2013-04-28), pages 1755-1766, XP055161609, ISSN: 0957-4530, DOI: 10.1007/s10856-013-4933-2 * abstract * * page 1756, right-hand column, paragraph 2-6 * * page 1757, left-hand column, paragraph 4 - right-hand column, paragraph 3 * * page 1758, right-hand column, paragraph 2 * | 1-3,7, 11-15 | INV. A61L27/38 A61L27/12 A61L27/46 C08L89/00 |
| A,D | WO 2010/007230 A1 (CENTRE NAT RECH SCIENT [FR]; BALAGUER THIERRY [FR]; ROCHET NATHALIE [F) 21 January 2010 (2010-01-21) * page 4, line 3 - page 11, line 23 * | 1-15 | |
| A | WO 2010/015619 A2 (UNIV DRESDEN TECH [DE]; HOFLACK BERNARD [DE]; SANCHEZ FERNANDEZ MARIA) 11 February 2010 (2010-02-11) * page 4, paragraph 5 - page 5, paragraph 7 * * page 6, paragraph 5 - page 7, paragraph 3 * | 1-15 | TECHNICAL FIELDS SEARCHED (IPC) A61L C08L |

-/--

The present search report has been drawn up for all claims

| Place of search | Date of completion of the search | Examiner |
|---|---|---|
| The Hague | 13 January 2015 | Lamers, Wolfram |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 14 30 6175 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DONG LEI ET AL: "Harnessing the power of macrophages/monocytes for enhanced bone tissue engineering", TRENDS IN BIOTECHNOLOGY, ELSEVIER PUBLICATIONS, CAMBRIDGE, GB, vol. 31, no. 6, 26 April 2013 (2013-04-26), pages 342-346, XP028550371, ISSN: 0167-7799, DOI: 10.1016/J.TIBTECH.2013.04.001 * page 343, left-hand column, paragraph 5 - right-hand column, paragraph 1 * * page 344, left-hand column, paragraph 1 - right-hand column, paragraph 1 * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 13 January 2015 | Lamers, Wolfram |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**  EP 14 30 6175

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2015

| Patent document cited in search report | Publication date | Patent family member(s) | | Publication date |
|---|---|---|---|---|
| WO 2010007230 A1 | 21-01-2010 | AU | 2009272594 A1 | 21-01-2010 |
| | | CA | 2728941 A1 | 21-01-2010 |
| | | CN | 102123740 A | 13-07-2011 |
| | | EP | 2307060 A1 | 13-04-2011 |
| | | FR | 2932686 A1 | 25-12-2009 |
| | | IL | 210181 A | 24-03-2013 |
| | | JP | 5456031 B2 | 26-03-2014 |
| | | JP | 2011525381 A | 22-09-2011 |
| | | KR | 20110105759 A | 27-09-2011 |
| | | RU | 2011102373 A | 27-07-2012 |
| | | US | 2011182950 A1 | 28-07-2011 |
| | | WO | 2010007230 A1 | 21-01-2010 |
| WO 2010015619 A2 | 11-02-2010 | DE | 102008036060 A1 | 25-02-2010 |
| | | EP | 2318518 A2 | 11-05-2011 |
| | | JP | 2011529756 A | 15-12-2011 |
| | | US | 2011189289 A1 | 04-08-2011 |
| | | WO | 2010015619 A2 | 11-02-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010007230 A

**Non-patent literature cited in the description**

- **T. BALAGUER ; F. BOUKHECHBA ; A. CLAVÉ ; S. BOUVET-GERBETTAZ ; C. TROJANI ; J. MICHIELS ; J. LAUGIER ; J. BOULER ; G. CARLE ; J. SCIMECA.** Biphasic Calcium Phosphate Microparticles for Bone Formation: Benefits of Combination with Blood Clot. *Tissue Engineering,* 2010, vol. 16 (11), 3495-3505
- **M. PITTENGER ; A. MACKAY ; S. BECK ; R. JAISWAL ; R. DOUGLAS ; J. MOSCA ; M. MOORMAN ; D. SIMONETTI ; S. CRAIG ; D. MARSHAK.** Multilineage potential of adult human mesenchymal stem cells. *Science,* 1999, vol. 284 (5411), 143-147
- **R. PIGNOLO ; M. KASSEM.** Circulating Osteogenic Cells: Implications for Injury, Repair, and Regeneration. *Journal of Bone and Mineral Research,* 2011, vol. 26 (8), 1685-1693
- **M. KUWANA ; Y. OKAZAKI ; H. KODAMA ; K. IZUMI ; H. YASUOKA ; Y. OGAWA ; Y. KAWAKAMI ; Y. IKEDA.** Human circulating CD14+ monocytes as a source of progenitors that exhibit mesenchymal cell differentiation. *Journal of Leukocyte Biology,* 2003, vol. 74, 833-845
- **C. MOULINE ; D. QUINCEY ; J. LAUGIER ; G. CARLE ; J. BOULER ; N. ROCHET ; J. SCIMECA.** Osteoclastic differentiation of mouse and human monocytes in a plasma clot/biphasic calcium phosphate microparticles composite. *European Cells and Materials,* 2010, vol. 20, 379-392
- **M. RABILLARD ; J. GRAND ; E. DALIBERT ; B. FELLAH ; O. GAUTHIER ; G. NIEBAUER.** Effects of autologous platelet rich plasma gel and calcium phosphate biomaterials on bone healing in an ulnar ostectomy model in dogs. *Vet Comp Orthop Traumatol,* 2009, vol. 22, 460-466
- **Z. ZHANG ; J. SHIVELY.** Acceleration of Bone Repair in NOD/SCID Mice by Human Monoosteophils, Novel LL-37-Activated Monocytes. *PLOS ONE,* 2013, vol. 8 (7
- **Z. ZHANG ; J. SHIVELY.** Generation of Novel Bone Forming Cells (Monoosteophils) from the Cathelicidin-Derived Peptide LL-37 Treated Monocytes. *PLOS ONE,* 2010, vol. 5 (11

- **L. DONG ; C. WANG.** Harnessing the power of macrophages/monocytes for enhanced bone tissue engineering. *Trends Biotechnol,* 2013, vol. 31 (6), 342-346
- **H. ZHANG ; Q. ZHU.** Preparation of porous hydroxyapatite with interconnected pore architecture. *J MaterSci Mater Med,* 2007, vol. 18 (9), 1825-1829
- **M. JARCHO.** Biomaterial aspects of calcium phosphates. Properties and applications. *Dent Clin North Am,* 1986, vol. 30 (1), 25-47
- **W. PICKL ; O. MAJDIC ; P. KOHL ; J. STÖCKL ; E. RIEDL ; C. SCHEINECKER ; C. BELLO-FERNANDEZ ; W. KNAPP.** Molecular and functional characteristics of dendritic cells generated from highly purified CD14+ peripheral blood monocytes. *J Immunol,* 1996, vol. 157 (9), 3850-3859
- **A. DE BAEY ; A. LANZAVECCHIA.** The role of aquaporins in dendritic cell macropinocytosis. *J Exp Med,* 2000, vol. 191 (4), 743-748
- **M. SALIO ; V. CERUNDOLO ; A. LANZAVECCHIA.** Dendritic cell maturation is induced by mycoplasma infection but not by necrotic cells. *Eur J Immunol,* 2000, vol. 30 (2), 705-708
- **S. EBNER ; S. HOFER ; V. NGUYEN ; C. FURHAPTER ; M. HEROLD ; P. FRITSCH ; C. HEUFLER ; N. ROMANI.** A novel role for IL-3: human monocytes cultured in the presence of IL-3 and IL-4 differentiate into dendritic cells that produce less IL-12 and shift Th cell responses toward a Th2 cytokine pattern. *J Immunol,* 2002, vol. 168 (12), 6199-6207
- *Blood,* 2003, vol. 102 (5), 1753-1763
- **M. MATSUMOTO ; K. FUNAMI ; M. TANABE ; H. OSHIUMI ; M. SHINGAI ; Y. SETO ; A. YAMAMOTO ; T. SEYA.** Subcellular localization of Toll-like receptor 3 in human dendritic cells. *J Immunol.,* 2003, vol. 171 (6), 3154-3162

- **P. HANLEY ; B. MUSSET ; V. RENIGUNTA ; S. LIMBERG ; A. DALPKE ; R. SUS ; K. HEEG ; R. PREISIG-MÜLLER ; J. DAUT.** Extracellular ATP induces oscillations of intracellular Ca2+ and membrane potential and promotes transcription of IL-6 in macrophages. *Proc Natl Acad Sci USA,* 2004, vol. 101 (25), 9479-9484
- **F. VERRECK ; T. DE BOER ; D. LANGENBERG ; M. HOEVE ; M. KRAMER ; E. VAISBERG ; R. KASTELEIN ; A. KOLK ; R. DE WAAL-MALEFYT ; T. OTTENHOFF.** Human IL-23-producing type 1 macrophages promote but IL-10-producing type 2 macrophages subvert immunity to (myco)bacteria. *Proc Natl Acad Sci USA,* 2004, vol. 101 (13), 4560-4565
- **E. RYAN ; A. MARSHALL ; D. MAGALETTI ; H. FLOYD ; K. DRAVES ; N. OLSON ; E. CLARK.** Dendritic cell-associated lectin-1: a novel dendritic cell-associated, C-type lectin-like molecule enhances T cell secretion of IL-4. *J Immunol,* 2002, vol. 169 (10), 5638-5648
- **S. VITALE ; A. SCHMID-ALLIANA ; V. BREUIL ; M. POMERANZ ; M. MILLET ; B. ROSSI ; H. SCHMID-ANTOMARCHI.** Soluble fractalkine prevents monocyte chemoattractant protein-1-induced monocyte migration via inhibition of stress-activated protein kinase 2/p38 and matrix metalloproteinase activities. *J Immunol,* 2004, vol. 172 (1), 585-592